# FASCICULE DE BREVET EUROPEEN

(11) **EP 2 246 091 B1**
(45) Date de publication et mention de la délivrance du brevet: **02.11.2011**
(21) Numéro de dépôt: 10154737.0
(22) Date de dépôt: 25.02.2010
(51) Int. Cl.: A61N 1/05

(54) **Sonde intracardiaque de stimulation ou de défibrillation du type à vis rétractable**
Intrakardialer Sensor zur Stimulation oder Defibrillation mit zurückziehbarer Schraube
Endocardiac stimulation or defibrillation probe with a retractable screw

(30) Priorité: 28.04.2009 FR 0952766
(43) Date de publication de la demande: 03.11.2010
(73) Titulaire: Sorin CRM SAS, 92541 Montrouge (FR)
(72) Inventeur: Ollivier, Jean-François, 91190, VILLIERS LE BACLE (FR)
(74) Mandataire: Dupuis-Latour, Dominique

(56) Documents cités:
- EP-A- 0 591 053
- EP-A- 1 331 021
- EP-A- 1 754 507
- EP-A- 1 774 986
- WO-A-2007/053065
- DE-A1- 3 712 082
- US-A- 4 106 512
- US-A- 5 531 780
- US-A- 5 571 157
- US-A- 5 837 006
- US-A1- 2004 068 299

## Description

L'invention concerne les sondes intracardiaques de stimulation ou de défibrillation.

Ces sondes, qui aboutissent dans une cavité du myocarde, permettent le recueil de signaux de dépolarisation pour la surveillance en continu du rythme cardiaque et si nécessaire l'application d'impulsions électriques de stimulation, de resynchronisation, de cardioversion et/ou de défibrillation. Ces sondes sont couplées à un générateur implanté, avec lequel elles forment un "dispositif médical implantable actif" tel que défini par la Directive 90/385/CEE du 20 juin 1990 du Conseil des communautés européennes.

L'invention concerne plus précisément les sondes pourvues à leur extrémité distale d'une vis rétractable permettant l'ancrage dans le tissu de l'endocarde au point de contact avec ce dernier.

De façon générale, une sonde de stimulation ou de défibrillation est constituée d'un "corps de sonde" formé d'une gaine (tube creux flexible) terminée à son extrémité distale par une "tête de sonde" portant l'électrode ou les électrodes destinées à venir en contact avec le myocarde, ainsi que la vis rétractable. Le corps de sonde est relié à son extrémité proximale à une fiche de connexion électrique raccordable à une tête de connecteur d'un stimulateur ou d'un défibrillateur, le tube du corps de sonde comportant un ou plusieurs conducteurs reliant électriquement les bornes de la fiche de connexion à l'électrode ou aux électrodes de la tête.

Dans le cas d'une sonde à vis rétractable, la tête de sonde est en outre pourvue d'un mécanisme permettant d'escamoter la vis de manière à protéger les parois de la veine, et éviter l'accrochage involontaire à la valve tricuspide ou à ses piliers, pendant l'introduction de la sonde jusqu'à ce que l'extrémité de la tête de sonde vienne en butée contre la paroi de l'endocarde. Le système à vis rétractable protège également la vis d'éventuelles déformations qui la rendraient inopérante.

Une fois la position atteinte, le chirurgien manipule alors la sonde de manière à déplacer la vis suivant un double mouvement simultané de translation axiale, pour déployer la vis hors du logement de la tête de sonde où elle se trouvait, et de rotation de cette vis pour réaliser son ancrage dans la paroi de l'endocarde.

La manipulation de la sonde est habituellement du type *pin-driven,* où le chirurgien maintient d'une main l'extrémité proximale, formant connecteur, du corps de sonde et fait tourner de l'autre main, directement ou par l'intermédiaire d'un outil, la fiche (*pin*) dépassant de cette extrémité proximale. Concrètement, la fiche est solidaire d'un conducteur axial s'étendant à l'intérieur du corps de sonde, ce conducteur étant libre en rotation et étant relié à son extrémité distale au mécanisme de sortie et d'entraînement de la vis.

Cette technique suppose bien entendu que la configuration des conducteurs intégrés au corps de sonde soit de type "configuration coaxiale", c'est-à-dire avec un conducteur central (utilisé pour transférer le couple permettant l'entraînement du mécanisme de déploiement de la vis) et un conducteur s'étendant en périphérie du corps de sonde.

Une variante de cette manipulation, obtenue non pas par rotation de la broche du connecteur mais par l'extrémité d'un mandrin introduit dans le corps de sonde, est décrite dans le EP 1 331 021 A1 (ELA Médical). Cette technique permet de disposer d'une fonction de limitation du couple d'entraînement de la vis, par vrillage de l'âme du mandrin. La limitation du couple d'entraînement de la vis permet en particulier d'éviter tout endommagement des tissus et/ou de la vis, ceci afin de rendre possible plusieurs procédures de fixation si les performances électriques sont insuffisantes. Une autre technique encore est proposée par le EP 0 591 053 A1 qui décrit une sonde actuellement commercialisée par ELA Médical sous la dénomination *Stelix* (marque déposée). Pour déployer la vis, le chirurgien introduit dans le corps de sonde un mandrin de type "mandrin-tournevis" dont l'extrémité distale présente une forme aplatie, apte à coopérer avec un organe homologue du mécanisme de déploiement de la vis à l'intérieur de la tête de sonde. Le déploiement est obtenu en maintenant fixe d'une main le mandrin-tournevis, et en faisant tourner de l'autre main le corps de sonde (par la partie proximale de la gaine) sur cinq à six tours complets. La tête de sonde est munie d'un système de limitation de couple qui minimise la contrainte de torsion lors du vissage : si le couple résistant lors du vissage dépasse un seuil donné, la vis n'est plus entraînée par la rotation du corps de sonde.

Ce système est efficace et sûr. Il est en outre réversible, le chirurgien pouvant dévisser la vis et la rétracter par une manoeuvre inverse de celle décrite. En revanche, la manoeuvre de déploiement par rotation du corps de sonde va à l'encontre de la manoeuvre usuelle *pin-driven* consistant, comme exposé plus haut, à maintenir fixe l'extrémité du corps de sonde et à faire tourner la broche d'extrémité du connecteur (ou la poignée d'un mandrin émergeant du corps de sonde au même endroit).

Un autre inconvénient, propre à tous les dispositifs à vis rétractable du type décrit ci-dessus, réside dans leur encombrement relativement important. Le diamètre standard de ces sondes est en effet de 7 French (1 French = 1/3 mm), nécessitant donc la mise en place d'un introducteur de 8 French. Cette valeur de 7 French correspond à une limite technique minimale qu'il est difficile et dangereux de dépasser avec les sondes du type à "configuration coaxiale" exposé plus haut. En effet, la fiabilité de la sonde passe par des épaisseurs minimales d'isolant et de conducteur pour le corps de sonde. De plus, le mécanisme rétractable doit lui aussi être techniquement fabricable si l'on souhaite conserver une configuration de type "isodiamètre", c'est-à-dire qui présente le même diamètre sur toute la longueur de la partie distale destinée à être implantée dans le réseau veineux, précisément pour faciliter cette implantation (ainsi qu'une explantation ultérieure). Ceci signifie que la surface extérieure de la sonde, y compris à l'endroit des électrodes et du mécanisme de déploiement de la vis, ne présente aucun saut de diamètre. Ces contraintes rendent délicate la réalisation de ces sondes, notamment en raison de la nécessité de réaliser une liaison électrique fiable entre les conducteurs intérieur et extérieur et les électrodes correspondantes de la tête de sonde. Pour diminuer le diamètre des sondes, une configuration différente des conducteurs de liaison a été proposée, configuration connue sous les dénominations de "coradiale" ou "à fils isolés" ou "à conducteurs isolés". Une sonde réalisée selon cette technologie est décrite par exemple dans le US 5 571 157 A.

Dans cette configuration coradiale, les deux (ou plus) conducteurs sont des conducteurs isolés bobinés tous deux côte à côte dans une région de la paroi périphérique de la gaine du corps de sonde, en formant un bobinage de rayon unique (d'où la dénomination "coradiale"), donc sur une seule épaisseur, à la différence des configurations coaxiales nécessitant deux (ou plus) épaisseurs pour loger les différents conducteurs.

Dans la mesure où les conducteurs sont tous disposés sur une seule couche, il est possible de réduire le diamètre standard jusqu'à 4,8 French (la sonde étant implantable avec un introducteur de 5 French), donc aussi faible que celui d'une sonde monopolaire, mais avec les fonctionnalités d'une sonde bipolaire ou multipolaire. Le diamètre réduit rend la sonde beaucoup plus manoeuvrable dans le réseau veineux lors de sa mise en place, et il est également possible de prévoir des systèmes avec deux sondes conjointement implantées dans le ventricule droit.

Si l'on souhaite équiper une telle sonde de type coradiale d'un mécanisme de fixation à vis déployable, la technique de manoeuvre *pin-driven* n'est a *priori* pas applicable. En effet, il paraît difficile de faire tourner les conducteurs l'un par rapport à l'autre (même si cette possibilité est envisagée par le US 5 716 390 A), du fait des contraintes de fiabilité des connexions électriques aux deux extrémités de la sonde. D'autre part, la capacité limitée des conducteurs bobinés à transmettre un couple réduit l'efficacité de ce moyen de déploiement de la vis d'ancrage.

L'un des buts de l'invention est de proposer une sonde bipolaire ou multipolaire à vis d'ancrage rétractable présentant l'ensemble des caractéristiques suivantes :
- mécanisme de déploiement de la sonde permettant d'utiliser une configuration de type coradiale, avec un diamètre standard pouvant atteindre 4,8 French ;
- mécanisme de déploiement de la sonde permettant une manoeuvre de type *pin-driven* conforme aux habitues des chirurgiens, avantageusement sans outillage additionnel ;
- transmission efficace du couple, avec une réponse de type "1 pour 1", c'est-à-dire une transmission procurant une réponse immédiate dans un sens et dans l'autre, sans hystérésis, contrairement aux conceptions classiques impliquant le conducteur interne comme élément de transmission, qui nécessite habituellement une rotation de quatre ou cinq tours depuis l'extrémité proximale avant d'obtenir une réponse à l'extrémité distale ;
- protection contre le survissage (fonction *overtorque*), c'est-à-dire que si par exemple trois à quatre tours sont nécessaires pour le déploiement et l'ancrage de la vis, un nombre plus élevé de tours sera sans incidence, grâce à un système de débrayage automatique intervenant dès que le couple résistant sur la vis d'ancrage dépasse une limite calibrée ;
- possibilité d'extraction aisée, par une manoeuvre inverse de celle exécutée pour le déploiement et l'ancrage de la sonde ;
- grande manoeuvrabilité dans le réseau veineux, par minimisation de la longueur de la partie distale rigide logeant la vis d'ancrage et son mécanisme de déploiement : la réduction des dimensions de cette partie rigide (typiquement à une longueur ne dépassant pas 12 mm) permet un meilleur avancement de la tête de sonde au moment de son introduction dans le réseau veineux ; de plus, une partie distale rigide trop longue qui déborderait dans l'oreillette ne serait pas satisfaisant sur le long terme ;
- performances électriques élevées, notamment grâce à une faible surface de l'électrode de stimulation distale (typiquement non supérieure à 2 mm², alors qu'avec les configurations coaxiales disponibles actuellement, cette surface est rarement inférieure à 4 mm²) et une faible distance inter-électrodes (typiquement non supérieure à 10 mm) ;
- configuration intégralement isodiamètre, sur toute la longueur de la sonde y compris la partie distale logeant la vis d'ancrage et son mécanisme de déploiement ;
- présence de marqueurs radio-opaques pour le contrôle du déploiement de la vis d'ancrage sous amplificateur de brillance ;
- possibilité de "mapping", c'est-à-dire de recherche de la position optimale de la sonde avant déploiement de la vis, sous amplificateur de brillance, jusqu'à trouver la position présentant la meilleure réponse à la stimulation électrique où la sonde pourra être solidarisée à l'endocarde par le déploiement de la vis d'ancrage, ce mapping étant effectué par l'intermédiaire (i) d'une électrode définitive (celle de la sonde), et (ii) avec la surface de stimulation définitive ;
- possibilité d'introduire un mandrin de guidage dans le corps de sonde qui vienne en butée, côté distal, en un point situé le plus proche possible de l'extrémité de la tête de sonde, de manière à pouvoir exercer la poussée le plus près possible de cette extrémité ;
- fabrication à partir de matériaux préexistants et avec des techniques de "process" éprouvées, sans contrainte technologique additionnelle.

L'invention propose à cet effet une sonde intracardiaque de stimulation ou de défibrillation du type sonde à vis déployable, connue par exemple d'après le EP 0 591 053 A1 précité.

Une telle sonde comprend une gaine creuse souple terminée à son extrémité distale par une tête de sonde et à son extrémité proximale par un connecteur comprenant au moins un broche de liaison à une électrode de la tête de sonde. La tête de sonde comporte : un corps extérieur tubulaire ; au moins une électrode de stimulation ou de défibrillation ; une vis d'ancrage mobile à l'intérieur du corps tubulaire entre une position rétractée et une position déployée, cette vis d'ancrage étant montée à l'extrémité distale d'un équipage mobile en translation et en rotation à l'intérieur du corps tubulaire en suivant un mouvement hélicoïdal ; et un mécanisme de manoeuvre, comprenant des moyens de couplage à l'équipage mobile, pour commander le déplacement de l'équipage mobile de la position rétractée vers la position déployée et inversement de la position déployée vers la position rétractée. La tête de sonde est apte à être entraînée en rotation pour permettre l'entraînement simultané de la vis et son ancrage par pénétration dans la paroi de l'endocarde. La sonde comporte en outre au moins un fil conducteur s'étendant sur toute la longueur de la gaine et reliant l'électrode, ou une électrode correspondante, à la broche de liaison du connecteur à l'extrémité proximale.

De façon caractéristique de l'invention, le conducteur, ou les conducteurs, sont des conducteurs isolés bobinés dans une région de la paroi périphérique de la gaine creuse selon une configuration de type coradiale. D'autre part, les moyens de couplage du mécanisme de manoeuvre sont des moyens de couplage de l'équipage mobile au corps tubulaire comprenant un guide hélicoïdal porté par l'équipage mobile. Ce guide hélicoïdal est mobile en translation et en rotation à l'intérieur du corps tubulaire, et étant apte à transformer un mouvement de rotation imprimé au corps tubulaire par une rotation de la gaine creuse transmise depuis l'extrémité proximale, en un mouvement de l'équipage mobile de la position rétractée vers la position déployée, et *vice versa.*

Selon diverses caractéristiques subsidiaires avantageuses :
- le sens du pas du guide hélicoïdal est inverse de celui de la vis d'ancrage ;
- les moyens de couplage comprennent un doigt de couplage solidaire du corps tubulaire et faisant saillie entre deux spires successives du guide hélicoïdal ;
- l'équipage mobile comporte deux flasques d'extrémité, et le guide hélicoïdal est intercalé entre ces deux flasques ;
- le guide hélicoïdal comprend une extrémité libre, et une extrémité opposée pourvue de moyens de solidarisation au flasque en vis-à-vis ;
- le guide hélicoïdal est un élément élastiquement compressible, configuré de sorte que lors du mouvement de déploiement, en fin de course du doigt de couplage, le couple de réaction produit entre la vis d'ancrage et le corps tubulaire entraîne une compression progressive du guide hélicoïdal, puis un débrayage des moyens de couplage, avec par voie de conséquence une limitation du couple transmis à la vis par la rotation du corps tubulaire, même en cas de poursuite de cette rotation ;
- le guide hélicoïdal comprend une extrémité libre présentant un méplat tourné vers le flasque en vis-à-vis, de manière à pincer le doigt de couplage entre ce flasque et le méplat lors de la compression du guide hélicoïdal ;
- le guide hélicoïdal comprend à son extrémité distale une butée de limitation de la course du doigt de couplage le long du guide hélicoïdal ;
- la tête de sonde comprend en outre un tube central de guidage axial de l'équipage mobile, solidaire en rotation du corps tubulaire et pouvant comporter à son extrémité libre distale une cartouche de libération d'un principe actif au point d'ancrage de la tête de sonde.

On va maintenant décrire un exemple de réalisation de l'invention, en référence aux dessins annexés où les mêmes références numériques désignent d'une figure à l'autre des éléments identiques ou fonctionnellement semblables.
Les Figures 1a et 1b représentent, respectivement en coupe longitudinale par un plan axial et en vue perspective partiellement écorchée, la tête d'une sonde selon l'invention, dans une configuration où la vis d'ancrage est en position rétractée.
La Figure 2 illustre, isolément, le guide hélicoïdal du mécanisme de déploiement de la vis d'ancrage.
La Figure 3 représente la sonde de l'invention à l'extrémité opposée, côté proximal où se trouve le connecteur.
Les Figures 4a et 4b sont homologues des Figures 1 a et 1b, dans une configuration où la vis d'ancrage est en fin de déploiement.
Les Figures 5a et 5b sont homologues des Figures 4a et 4b, dans une configuration où la vis d'ancrage, entièrement déployée, est entraînée en rotation par le corps de sonde pour la visser dans les tissus de l'endocarde.

On va maintenant décrire un exemple de réalisation de la sonde de l'invention.

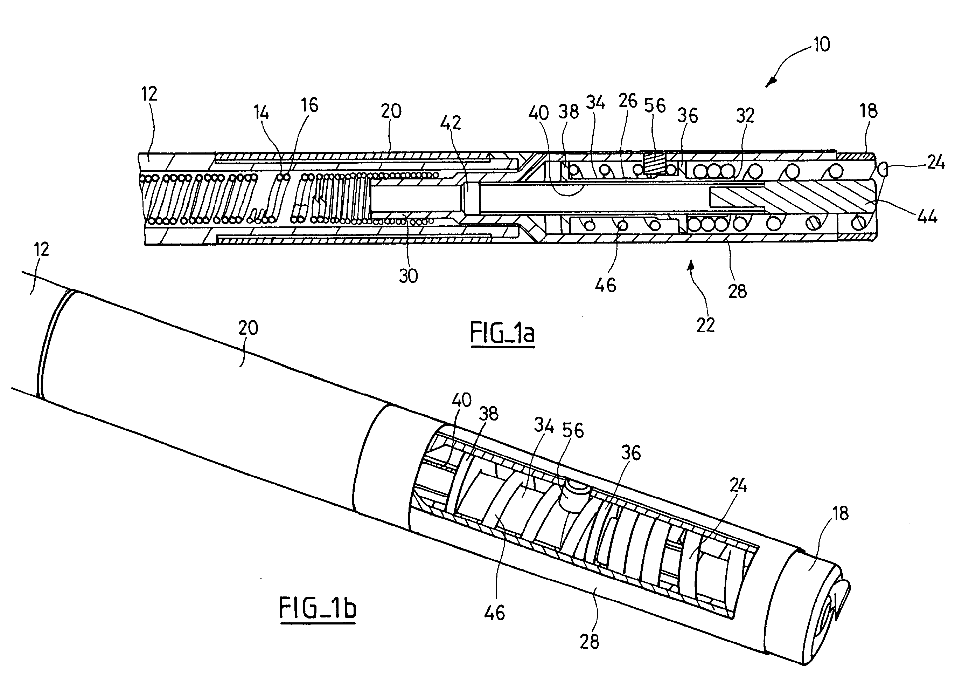

Sur les Figures 1 a et 1b, on a représenté une tête de sonde 10 d'une sonde du type à vis rétractable, avec la vis d'ancrage en position rétractée.

Cette tête de sonde 10 est montée à l'extrémité d'une gaine 12 avec laquelle elle constitue le corps de sonde. La gaine 12 a la forme d'un tube creux flexible incorporant deux (ou plus) conducteurs électriques 14, 16 reliés à des électrodes respectives 18, 20 (sur les dessins, on a représenté un système d'enroulement colinéaire à double fil, avec deux fils indépendants connectés à chaque électrode, par sécurité). L'électrode 18 est une électrode annulaire distale ou *tip,* et l'électrode 20 est une électrode proximale ou *ring.* La tête de sonde comporte en outre un mécanisme 22 permettant de déployer une vis 24 destinée à venir s'ancrer dans la paroi de l'endocarde, pour assurer une liaison mécanique avec le tissu du myocarde et empêcher tout déplacement ou délogement de la tête de sonde 12 une fois celle-ci mise en place.

La vis d'ancrage 24 est solidaire d'un équipage mobile 26 logé dans un corps extérieur tubulaire 28 du corps de sonde. Le corps extérieur tubulaire 28 est solidarisé à la gaine 12 par exemple par collage, et se termine en partie arrière par une queue 30 reliée électriquement et mécaniquement au conducteur 14 de manière à assurer la continuité électrique depuis ce conducteur jusqu'à l'électrode distale 18 située à l'extrémité opposée.

Le corps extérieur tubulaire 28 est avantageusement réalisé en titane, et il est revêtu extérieurement d'une couche isolante, par exemple un dépôt de parylène, de même que la vis d'ancrage 24, qui est une vis isolée. Le corps extérieur 28 en titane est radio-transparent, ce qui permet de suivre la mise en place de la tête de sonde sous amplificateur de brillance avec les marqueurs radio-opaques constitués des électrodes 18 et 20, réalisés en platine iridié opaque aux rayons. Le marqueur radio-opaque de sortie de vis est constitué, selon une technique courante, par les deux masses radio-opaques constituées par l'électrode distale en platine iridié et par le paquet de spires contigües formant la base de la vis d'ancrage (également en platine iridié), les deux masses se rapprochant lorsque la vis est sortie.

On notera qu'il est possible de donner à l'électrode 18 une surface de contact réduite, de l'ordre de 2 mm². La compacité du mécanisme de déploiement de la sonde permet également de réduire la distance inter-électrodes entre électrodes distale 18 et proximale 20, à une valeur de l'ordre de 10 mm seulement. Une autre conséquence est la réduction de la longueur de la partie rigide de la tête de sonde, à une valeur de l'ordre de 12 mm pour la longueur du corps tubulaire 28, ce qui facilite l'introduction et la progression de la sonde dans le réseau veineux. En vue diamétrale, la dimension hors-tout de la tête de sonde peut être réduite à une valeur de 4,8 French, la même que celle de la gaine souple 12 (configuration isodiamètre), permettant une mise en place avec un introducteur de diamètre 5 French seulement.

On va maintenant décrire plus en détail la structure du mécanisme 22 de déploiement de la vise d'ancrage.

L'équipage mobile 26 comporte une partie distale en saillie 32 sur laquelle est solidarisée, par exemple par soudage, la vis d'ancrage 24, de manière que celle-ci puisse suivre les déplacements en translation et en rotation de l'équipage mobile 26. La partie centrale 34 de l'équipage mobile se présente sous une forme tubulaire, délimitée par deux flasques d'extrémité 36, 38 en forme de disques plats s'étendant dans des plans radiaux parallèles respectifs.

L'équipage mobile 26 coulisse sur un tube central de guidage axial 40 solidarisé dans sa région arrière en 42 avec le corps extérieur tubulaire 28. On notera que, lorsqu'un mandrin de guidage est introduit dans la lumière centrale du corps de sonde, l'extrémité de ce mandrin vient buter contre la région proximale 42 du tube de guidage. À son autre extrémité, distale, le tube de guidage 40 est avantageusement pourvu d'une cartouche 44 de libération d'un principe actif dans la région de contact de l'électrode distale 18 avec la paroi du myocarde.

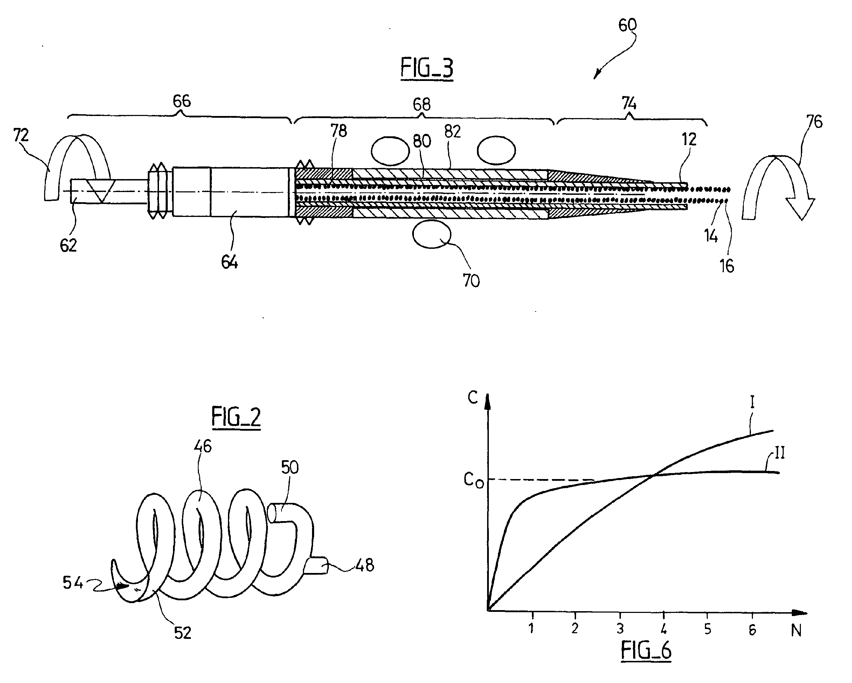

Dans la région centrale 34 comprise entre les flasques 36 et 38, l'équipage mobile 26 porte un élément 46 formant guide hélicoïdal, illustré isolément sur la Figure 2. Cet élément est réalisé en un matériau élastique et se présente par exemple sous forme d'un fil enroulé en hélice sur trois à quatre spires de manière à présenter la forme d'un ressort hélicoïdal. L'extrémité distale (à droite sur la Figure 2) du guide hélicoïdal 46 est solidarisée à l'équipage mobile, par exemple au moyen d'un ergot 48 orienté axialement et s'étendant dans une fente homologue (non représentée) du flasque distal 36, ou encore par soudure laser au flasque 36. À cette même extrémité est par ailleurs prévu un élément 50 jouant le rôle de butée, par exemple formé d'un retour de l'extrémité du fil cylindrique constituant le guide hélicoïdal, dirigé axialement et venant s'interposer dans l'intervalle formé par la dernière spire du guide hélicoïdal 46.

L'extrémité opposée, proximale 52 du guide hélicoïdal 46 est, à la différence de l'extrémité distale, une extrémité libre pourvue d'un méplat 54 s'étendant dans un plan sensiblement parallèle au plan du flasque proximal 38. Le guide 46 pourra donc être comprimé à la manière d'un ressort par une pression exercée sur son extrémité libre 52.

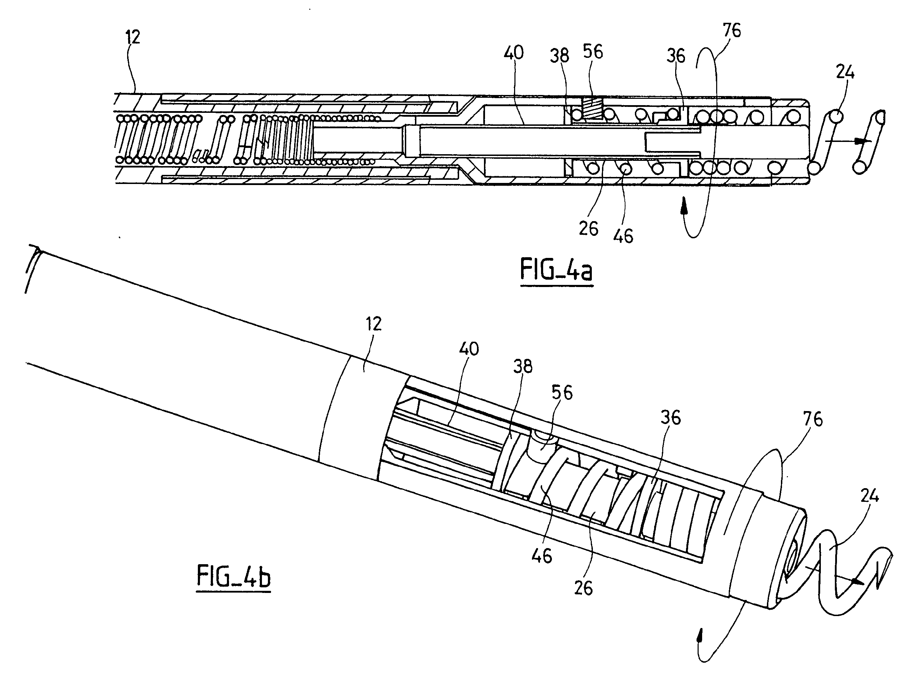

Le mécanisme de déploiement de la vis d'ancrage 22 comprend enfin un doigt de couplage 56 en forme d'élément cylindrique, solidaire du corps extérieur tubulaire 28 et dirigé radialement vers l'intérieur de celui-ci. La dimension de ce doigt est choisie pour que celui-ci vienne se placer dans l'intervalle compris entre deux spires successives du guide hélicoïdal 46, comme on peut le voir sur les Figures 1 a et 1 b. La fonction de ce doigt 56 est de transformer un mouvement (absolu) de rotation imprimé au corps de sonde, et donc au corps extérieur tubulaire 28, en un mouvement (relatif) de translation axiale de l'équipage mobile 26 par rapport à ce corps extérieur tubulaire 28 via le guide hélicoïdal 46, cette translation de l'équipage mobile entraînant le déploiement de la vis d'ancrage 24 hors du corps extérieur tubulaire 28. Les Figures 4a et 4b illustrent une telle configuration où la vis d'ancrage est en fin de déploiement.

On va maintenant décrire l'extrémité proximale 60 de la sonde de l'invention, côté connecteur, en référence à la Figure 3.

Cette extrémité 60 se présente extérieurement sous forme d'une connecteur standard, par exemple un connecteur normalisé IS-1, avec deux contacts 62, 64 reliés aux conducteurs respectifs 14, 16 incorporés au corps de sonde. Le contact 62 se présente sous forme d'une broche axiale, et il est relié via le conducteur 14 à l'électrode distale 18 située à l'autre extrémité de la sonde. Le contact 64 est un contact périphérique, relié quant à lui via le conducteur 16 à l'électrode proximale 20 de la tête de sonde.

La région 66 portant les contacts 62, 64 est prolongée côté distal par une région 68 dénommée capuche arrière, qui est désolidarisée en rotation de la région 66 portant les contacts. La zone de préhension fixe 68 ainsi formée par la capuche arrière peut être tenue d'une main entre les doigts du chirurgien (doigts schématisés en 70), tandis que de l'autre main celui-ci fait tourner la broche 62 (flèche 72), directement ou par l'intermédiaire d'un outil approprié. Cette rotation est transmise au corps de sonde (flèche 76), c'est-à-dire à la région 74 qui s'étend au-delà de la capuche arrière, sur toute l'étendue du corps de sonde jusqu'à la tête de sonde située à l'autre extrémité. Cette manoeuvre est une manoeuvre classique de type *pin-driven* permettant, par rotation de la broche (*pin*) 62, d'imprimer au corps de sonde dans son entier une rotation, transmise jusqu'à la tête de sonde située à l'autre extrémité. La capuche arrière, fixe pendant cette rotation, permet le maintien axial de la sonde par le chirurgien et assure la précision de l'entraînement en rotation de la broche 62. La zone de préhension fixe 68 de la capuche arrière comprend à cet effet sur la partie 78 prolongeant la gaine 12 du corps de sonde un élément de glissement 80, par exemple un tube en PTFE, pourvu d'un revêtement extérieur 82 facilitant sa préhension par les doigts du chirurgien, par exemple un revêtement en silicone.

On va maintenant expliciter la manière dont le chirurgien réalise l'implantation de la sonde que l'on vient de décrire.

Cette mise en oeuvre est basée sur le principe consistant à utiliser le corps de sonde tout entier (y compris, et surtout, l'isolant en polyuréthane de la gaine souple 12) pour transmettre le couple de manoeuvre depuis l'extrémité proximale jusqu'à l'extrémité distale, de la même manière que pour une sonde à vis fixe - mais en utilisant la même procédure de manipulation qu'une sonde à vis rétractable de type *pin-driven.* En effet, la manipulation reste pour le chirurgien comparable à ce dont il a l'habitude, avec l'enchaînement des étapes suivantes :
- mise en place d'un mandrin, préformé ou non, dans la lumière interne du corps de sonde ;
- introduction de la sonde jusqu'à la cavité cardiaque souhaitée par l'intermédiaire du réseau veineux ;
- mise en place éventuelle d'un outil de manipulation de la broche 62 du connecteur ;
- maintien, d'une main, de la capuche arrière du connecteur (zone de préhension 68) ;
- placement de l'extrémité de la sonde contre la paroi cardiaque et "harponnage" de la vis d'ancrage 24, la partie légèrement saillante de la vis (de l'ordre de 0,3 mm) venant piquer le tissu du myocarde et immobiliser en rotation la sonde. À ce stade, le chirurgien peut éventuellement procéder à un *mapping* pour évaluer la réponse du tissu avant de percer celui-ci ;
- rotation de la broche 62 dans le sens horaire, de manière à provoquer le déploiement de la vis d'ancrage 24 puis le vissage de celle-ci dans la paroi de l'endocarde ;
- recul du mandrin ;
- test de la fixation, par exemple par une légère traction.

Le mécanisme 22 de déploiement de la vis d'ancrage fonctionne de la manière suivante.

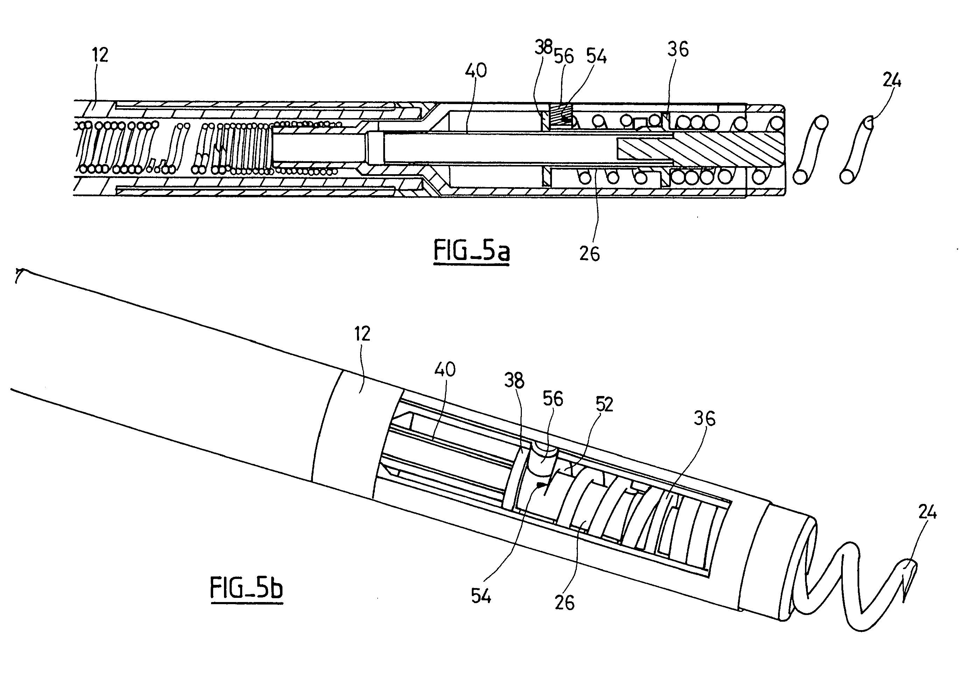

Une fois réalisé le harponnage de la vis d'ancrage 24 avec *mapping* éventuel par l'électrode distale 18 (on notera que ce *mapping* est effectué avec une électrode définitive et avec la surface de stimulation définitive, donc dans les meilleures conditions pour le choix du meilleur site de stimulation), la vis d'ancrage se trouve immobilisée en rotation. De ce fait, la rotation horaire du corps de sonde va entraîner, par réaction, la translation axiale vers l'avant de l'équipage mobile 26 via le doigt de couplage 56 et le guide hélicoïdal 46, dont le sens du pas est inversé par rapport à celui de la vis d'ancrage 24 et qui glisse sur le doigt de couplage 56. La vis d'ancrage 24 se dégage progressivement du boîtier, jusqu'à ce que le doigt d'entraînement 56 arrive à l'extrémité du guide hélicoïdal 46, dans une configuration correspondant à celle illustrée sur les Figures 4a et 4b. La poursuite de la rotation du corps de sonde, et donc de celle du doigt de couplage 56, va commencer à comprimer le guide hélicoïdal 46 du fait de l'élasticité de celui-ci, et le doigt de couplage 56 va se trouver bloqué dans l'"étau" formé entre le méplat 54 et le flasque proximal 38 situé en vis-à-vis. Cette configuration d"'embrayage" correspond à celle illustrée sur les Figures 5a et 5b.

L'équipage mobile 26, et donc la vis d'ancrage 24, va ainsi se trouver temporairement solidarisé par cette liaison de type embrayage, et via le guide hélicoïdal 46, au doigt de couplage 56 et donc au corps de sonde. La vis d'ancrage 24, déployée, va donc suivre en rotation le mouvement de rotation du corps de sonde que le chirurgien continue à imprimer à l'extrémité proximale.

La poursuite de cette rotation du corps de sonde aura pour effet le vissage de la vis d'ancrage 24 dans le tissu du myocarde, jusqu'à ce que l'élément de stimulation 18 vienne en contact avec la paroi de l'endocarde.

Ce contact va produire par réaction sur la vis d'ancrage un couple résistant accru, couple transmis au système d'embrayage. Lorsque ce couple dépassera la limite calibrée par la compression du guide hélicoïdal 46, le doigt de couplage 56 se libèrera de l'étau d'embrayage.

Toute rotation complémentaire du corps de sonde reproduira le même phénomène, il n'y aura donc pas de risque d'abimer les tissus ni même de perforer la paroi par un vissage excessif (risque de "carottage" produit par un survissage).

La Figure 6 illustre la caractéristique du couple transmis par le corps de sonde en fonction du nombre de tours imprimés à la broche 62 par le chirurgien, pour une sonde conventionnelle (caractéristique I) et pour la sonde de l'invention (caractéristique II). On notera la transmission très efficace du couple, par le corps de sonde (conducteurs et surtout isolant polyuréthanne) et par le mécanisme de déploiement de l'invention, avec une réponse quasiment de type "un pour un". Le couple atteint en effet très rapidement une valeur standard (caractéristique II), contrairement aux sondes classiques impliquant le conducteur interne comme élément de transmission, pour lesquelles il est nécessaire d'imprimer plusieurs tours à la broche avant de ressentir une réponse (caractéristique I).

En outre, le système de débrayage automatique, calibré à un couple Cₒ de l'ordre de 0,05 à 0,20 N.cm par exemple, assure une sécurité absolue et procure une très grande tolérance sur le nombre de tours à effectuer pour garantir une bonne fixation. En effet, même si trois à quatre tours suffisent, il n'y a aucune contre-indication si le chirurgien souhaite faire dix, vingt voire même trente tours. Le chirurgien ne ressentira pas de résistance, il percevra simplement à chaque débrayage un cliquetis lui confirmant que la vis d'ancrage a bien été déployée et vissée dans le tissu du myocarde - cette manoeuvre étant en outre obtenue sans aucun changement des habitudes liées à l'utilisation de sondes traditionnelles quelles qu'elles soient.

Une rotation en sens inverse du corps de sonde aura pour effet le réengrènement du doigt de couplage 56 dans les spires du guide hélicoïdal 46. Le doigt de couplage 56 suivra les spires jusqu'à venir buter contre l'organe 50 de limitation de course (Figure 2) assurant la venue en prise et l'accouplement en rotation du corps de sonde et de la vis d'ancrage. La poursuite de cette rotation en sens inverse aura pour effet de dévisser la vis d'ancrage 24 hors du tissu du myocarde. En fin de manoeuvre, l'ensemble sera prêt pour un repositionnement, après que le chirurgien ait légèrement tiré la sonde pour vérifier qu'elle est bien détachée de la paroi.

## Revendications

1. Une sonde intracardiaque de stimulation ou de défibrillation du type sonde à vis déployable, cette sonde comprenant une gaine creuse souple (12) terminée à son extrémité distale par une tête de sonde (10) et à son extrémité proximale par un connecteur (66) comprenant au moins un broche (62) de liaison à une électrode (18) de la tête de sonde,
la tête de sonde comportant :
- un corps extérieur tubulaire (28) ;
- au moins une électrode (18, 20) de stimulation ou de défibrillation ;
- une vis d'ancrage (24) mobile à l'intérieur du corps tubulaire entre une position rétractée et une position déployée, cette vis d'ancrage étant montée à l'extrémité distale d'un équipage (26) mobile en translation et en rotation à l'intérieur du corps tubulaire en suivant un mouvement hélicoïdal ; et
- un mécanisme de manoeuvre (22), comprenant des moyens de couplage à l'équipage mobile, pour commander le déplacement de l'équipage mobile de la position rétractée vers la position déployée et inversement de la position déployée vers la position rétractée,
la tête de sonde étant apte à être entraînée en rotation pour permettre l'entraînement simultané de la vis et son ancrage par pénétration dans la paroi de l'endocarde,
la sonde comportant en outre au moins un fil conducteur (14, 16) s'étendant sur toute la longueur de la gaine et reliant l'électrode, ou une électrode correspondante (18), à ladite au moins une broche de liaison (62) du connecteur (66) à l'extrémité proximale,
sonde ***caractérisée en ce que*** :
- le conducteur, ou lesdits conducteurs (20, 22), sont des conducteurs isolés bobinés dans une région de la paroi périphérique de la gaine creuse (12) selon une configuration de type coradiale, et
- lesdits moyens de couplage du mécanisme de manoeuvre sont des moyens de couplage de l'équipage mobile (26) au corps tubulaire (28) comprenant un guide hélicoïdal (46) porté par l'équipage mobile,
ce guide hélicoïdal étant mobile en translation et en rotation à l'intérieur du corps tubulaire, et étant apte à transformer un mouvement de rotation imprimé au corps tubulaire par une rotation de la gaine creuse transmise depuis l'extrémité proximale, en un mouvement de l'équipage mobile (26) de la position rétractée vers la position déployée, et *vice versa.*

2. La sonde intracardiaque de la revendication 1, dans laquelle le sens du pas du guide hélicoïdal (46) est inverse de celui de la vis d'ancrage (24).

3. La sonde intracardiaque de la revendication 1, dans laquelle lesdits moyens de couplage comprennent un doigt de couplage (56) solidaire du corps tubulaire (28) et faisant saillie entre deux spires successives du guide hélicoïdal (46).

4. La sonde intracardiaque de la revendication 3, dans laquelle l'équipage mobile (26) comporte deux flasques d'extrémité (36, 38), et le guide hélicoïdal (46) est intercalé entre ces deux flasques.

5. La sonde intracardiaque de la revendication 4, dans laquelle le guide hélicoïdal (46) comprend une extrémité libre (52), et une extrémité opposée pourvue de moyens (48) de solidarisation au flasque(36) en vis-à-vis.

6. La sonde intracardiaque de la revendication 4, dans laquelle le guide hélicoïdal (46) est un élément élastiquement compressible, configuré de sorte que lors du mouvement de déploiement, en fin de course du doigt de couplage (56), le couple de réaction produit entre la vis d'ancrage et le corps tubulaire entraîne une compression progressive du guide hélicoïdal, puis un débrayage des moyens de couplage, avec par voie de conséquence une limitation du couple transmis à la vis par la rotation du corps tubulaire, même en cas de poursuite de cette rotation.

7. La sonde intracardiaque de la revendication 6, dans laquelle le guide hélicoïdal (46) comprend une extrémité libre (52) présentant un méplat (54) tourné vers le flasque (38) en vis-à-vis, de manière à pincer le doigt de couplage (56) entre ce flasque et le méplat lors de la compression du guide hélicoïdal.

8. La sonde intracardiaque de la revendication 1, dans laquelle le guide hélicoïdal (46) comprend à son extrémité distale une butée (50) de limitation de la course du doigt de couplage (36) le long du guide hélicoïdal.

9. La sonde intracardiaque de la revendication 1, dans laquelle la tête de sonde (10) comprend en outre un tube central (40) de guidage axial de l'équipage mobile (26).

10. La sonde intracardiaque de la revendication 9, dans laquelle le tube central de guidage axial (40) est solidaire en rotation du corps tubulaire (28).

11. La sonde intracardiaque de la revendication 1, dans laquelle le tube central de guidage axial (40) comporte à son extrémité libre distale une cartouche de libération d'un principe actif au point d'ancrage de la tête de sonde.

## Claims

1. Endocardiac stimulation or defibrillation probe of the probe with extendable screw type, this probe comprising a flexible hollow sheath (12) terminated at its distal end by a probe head (10) and at its proximal end by a connector (66) comprising at least one pin (62) linking to an electrode (18) of the probe head, the probe head comprising:
- a tubular outer body (28);
- at least one stimulation or defibrillation electrode (18, 20);
- an anchoring screw (24) that can move inside the tubular body between a retracted position and an extended position, this anchoring screw being mounted at the distal end of an element (26) that can move translationally and rotationally inside the tubular body by a helical movement; and
- an operating mechanism (22), comprising means for coupling to the mobile element, to control the displacement of the mobile element from the retracted position to the extended position and, conversely, from the extended position to the retracted position,
the probe head being able to be driven in rotation to allow for the simultaneous driving of the screw and its anchoring by penetration into the wall of the endocardium,
the probe also comprising at least one conductive wire (14, 16) extending over the entire length of the sheath and linking the electrode, or a corresponding electrode (18), to said at least one pin (62) linking the connector (66) to the proximal end,
the probe being ***characterized in that***:
- the conductor, or said conductors (20, 22), is/are insulated conductors wound in a region of the peripheral wall of the hollow sheath (12) according to a coradial type configuration, and
- said coupling means of the operating mechanism are means for coupling the mobile element (26) to the tubular body (28) comprising a helical guide (46) borne by the mobile element,
this helical guide being able to move in translation and in rotation inside the tubular body, and being able to convert a rotational movement communicated to the tubular body by a rotation of the hollow sheath transmitted from the proximal end into a movement of the mobile element (26) from the retracted position to the extended position, and *vice versa.*

2. Endocardiac probe according to Claim 1, in which the direction of the pitch of the helical guide (46) is the reverse of that of the anchoring screw (24).

3. Endocardiac probe according to Claim 1, in which said coupling means comprise a coupling finger (56) attached to the tubular body (28) and protruding between two successive turns of the helical guide (46).

4. Endocardiac probe according to Claim 3, in which the mobile element (26) comprises two end flanges (36, 38), and the helical guide (46) is inserted between these two flanges.

5. Endocardiac probe according to Claim 4, in which the helical guide (46) comprises a free end (52), and an opposite end provided with means (48) for attachment to the facing flange (36).

6. Endocardiac probe according to Claim 4, in which the helical guide (46) is an elastically compressible element, configured so that, in an extension movement, at the end of travel of the coupling finger (56), the reaction torque produced between the anchoring screw and the tubular body results in a progressive compression of the helical guide, then a disengaging of the coupling means, with a consequential limitation of the torque transmitted to the screw by the rotation of the tubular body, even if this rotation is continued.

7. Endocardiac probe according to Claim 6, in which the helical guide (46) comprises a free end (52) having a flat (54) facing towards the facing flange (38), so as to grip the coupling finger (56) between this flange and the flat when the helical guide is compressed.

8. Endocardiac probe according to Claim 1, in which the helical guide (46) comprises, at its distal end, an end stop (50) for limiting the travel of the coupling finger (36) along the helical guide.

9. Endocardiac probe according to Claim 1, in which the probe head (10) also comprises a central tube (40) for axially guiding the mobile element (26).

10. Endocardiac probe according to Claim 9, in which the axial guiding central tube (40) is attached in rotation to the tubular body (28).

11. Endocardiac probe according to Claim 1, in which the axial guiding central tube (40) comprises, at its distal free end, an active principle release cartridge at the anchoring point of the probe head.

## Patentansprüche

1. Intrakardiale Stimulations- oder Defibrillationssonde des Typs Sonde mit ausfahrbarer Schraube, wobei diese Sonde eine nachgiebige hohle Hülle (12) umfasst, die an ihrem distalen Ende in einem Sondenkopf (10) endet und an ihrem proximalen Ende in einem Leiter (66) endet, der wenigstens einen Anschluss (62) für die Verbindung mit einer Elektrode (18) des Sondenkopfes aufweist,
wobei der Sondenkopf Folgendes umfasst:
- einen rohrförmigen Außenkörper (28);
- wenigstens eine Stimulations- oder Defibrillationselektrode (18, 20);
- eine Verankerungsschraube (24), die in dem rohrförmigen Körper zwischen einer zurückgezogenen Position und einer ausgefahrenen Position beweglich ist, wobei diese Verankerungsschraube am distalen Ende einer Ausrüstung (26) montiert ist, die translatorisch und rotatorisch in dem rohrförmigen Körper in einer Schraubenlinienbewegung beweglich ist; und
- einen Betätigungsmechanismus (22), der Mittel für die Kopplung der beweglichen Ausrüstung umfasst, um die Verlagerung der beweglichen Ausrüstung aus der zurückgezogenen Position in die ausgefahrene Position und umgekehrt aus der ausgefahrenen Position in die zurückgezogene Position zu steuern,
wobei der Sondenkopf dazu ausgelegt ist, rotatorisch angetrieben zu werden, um den gleichzeitigen Antrieb der Schraube und ihre Verankerung durch Eindringen in die endokardiale Wand zu ermöglichen,
wobei die Sonde außerdem wenigstens einen Leiterdraht (14, 16) umfasst, der sich über die gesamte Länge der Hülle erstreckt und die Elektrode oder eine entsprechende Elektrode (18) mit dem wenigstens einen Verbindungsanschluss (62) des Verbinders (66) am proximalen Ende verbindet,
wobei die Sonde **dadurch gekennzeichnet ist, dass**:
- der Leiter oder die Leiter (20, 22) isolierte Leiter sind, die in einem Bereich der Umfangswand der hohlen Hülle (12) in einer Konfiguration des koradialen Typs gewickelt sind, und
- die Kopplungsmittel des Betätigungsmechanismus Mittel zum Koppeln der beweglichen Ausrüstung (26) mit dem rohrförmigen Körper (28) sind und eine schraubenlinienförmige Führung (46) aufweisen, die von der beweglichen Ausrüstung getragen wird,
wobei diese schraubenlinienförmige Führung translatorisch und rotatorisch in dem rohrförmigen Körper beweglich ist und eine Drehbewegung, die dem rohrförmigen Körper durch eine Drehung der hohlen Hülle auferlegt wird, die von seinem proximalen Ende übertragen wird, in eine Bewegung der beweglichen Ausrüstung (26) aus der zurückgezogenen Position in die ausgefahrene Position und umgekehrt umwandeln kann.

2. Intrakardiale Sonde nach Anspruch 1, wobei der Bewegungsrichtungssinn der schraubenlinienförmigen Führung (46) zu jenem der Verankerungsschraube (24) entgegengesetzt ist.

3. Intrakardiale Sonde nach Anspruch 1, wobei die Kopplungsmittel einen Kopplungszapfen (56) umfassen, der mit dem rohrförmigen Körper (28) fest verbunden ist und zwischen zwei aufeinander folgenden Windungen der schraubenlinienförmigen Führung (46) vorsteht.

4. Intrakardiale Sonde nach Anspruch 3, wobei die bewegliche Ausrüstung (26) zwei Stirnflansche (36, 38) umfasst und die schraubenlinienförmige Führung (46) zwischen diese zwei Flansche eingefügt ist.

5. Intrakardiale Sonde nach Anspruch 4, wobei die schraubenlinienförmige Führung (46) ein freies Ende (52) und ein gegenüberliegendes Ende, das mit Mitteln (48) für eine Befestigung gegenüber dem Flansch (36) versehen ist, umfasst.

6. Intrakardiale Sonde nach Anspruch 4, wobei die schraubenlinienförmige Führung (46) ein elastisch komprimierbares Element ist, das in der Weise konfiguriert ist, dass bei einer Ausfahrbewegung am Ende der Bahn des Kopplungszapfens (56) das Gegendrehmoment, das zwischen der Verankerungsschraube und dem rohrförmigen Körper erzeugt wird, eine progressive Kompression der schraubenlinienförmigen Führung und dann eine Entkopplung der Kopplungsmittel und folglich eine Begrenzung des durch die Drehung des rohrförmigen Körpers auf die Schraube übertragenen Drehmoments selbst bei einer Fortsetzung dieser Drehung zur Folge hat.

7. Intrakardiale Sonde nach Anspruch 6, wobei die schraubenlinienförmige Führung (46) ein freies Ende (52) umfasst, das eine zu dem gegenüber befindlichen Flansch (38) gewendete Abflachung (54) aufweist, derart, dass der Kopplungszapfen (56) zwischen diesen Flansch und diese Abflachung bei der Kompression der schraubenlinienförmigen Führung eindringt.

8. Intrakardiale Sonde nach Anspruch 1, wobei die schraubenlinienförmige Führung (46) an ihrem distalen Ende einen Anschlag (50) für die Begrenzung der Bahn des Kopplungszapfens (36) längs der schraubenlinienförmigen Führung umfasst.

9. Intrakardiale Sonde nach Anspruch 1, wobei der Sondenkopf (10) außerdem ein Mittelrohr (40) für die axiale Führung der beweglichen Ausrüstung (26) umfasst.

10. Intrakardiale Sonde nach Anspruch 9, wobei das axiale Führungsmittelrohr (40) mit dem rohrförmigen Körper (28) drehfest verbunden ist.

11. Intrakardiale Sonde nach Anspruch 1, wobei das axiale Führungsmittelrohr (40) an seinem distalen freien Ende eine Kartusche für die Freisetzung eines natürlichen Wirkstoffs am Verankerungspunkt des Sondenkopfes aufweist.
